(19)

**Europäisches Patentamt**

**European Patent Office**

**Office européen des brevets**

(11) **EP 2 767 829 A1**

(12) **EUROPÄISCHE PATENTANMELDUNG**

(43) Veröffentlichungstag:
20.08.2014 Patentblatt 2014/34

(21) Anmeldenummer: 13155658.1

(22) Anmeldetag: 18.02.2013

(51) Int Cl.:
*G01N 33/50* (2006.01)   *C07K 14/705* (2006.01)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(71) Anmelder: **Universität Wien**
**1010 Wien (AT)**

(72) Erfinder:
• **Somoza, Veronika**
**3400 Weidling (AT)**

• **Liszt, Kathrin Ingrid**
**7471 Rechnitz (AT)**
• **Köck, Elke**
**8820 Neumarkt (AT)**
• **Ley, Jakob**
**37603 Holzminden (DE)**

(74) Vertreter: **Sandmann, Wolfgang**
**Isarpatent**
**Friedrichstraße 31**
**DE-80801 München (DE)**

(54) **Verfahren zur Identifizierung von Bitterstoffen und bittermodulierenden Stoffen**

(57) Vorgeschlagen wird ein in vitro-Verfahren zur Identifizierung von Bitterstoffen und bittermodulierenden Stoffen, bei dem man Testsubstanzen mit isolierten Magenzellen oder Magentumor-Zelllinien in Kontakt bringt und die dadurch hervorgerufene Änderung des intrazellularen pH-Wertes und/oder die zellulare Protonensekretion bestimmt.

EP 2 767 829 A1

**Beschreibung**

**Gebiet der Erfindung**

Die Erfindung betrifft ein Verfahren zur Identifizierung von Bitterstoffen und bittermodulierenden, insbesondere bittermaskierenden (Bitter-Antagonisten) und bitterverstärkenden (Bitter-Agonisten), durch Änderung des interzellularen pH-Wertes bzw. der Protonensekretion.

**Stand der Technik**

Nahrungs- und Genussmittel enthalten häufig eine Vielzahl verschiedener Bitterstoffe (Bitter-Agonisten), die zwar einerseits in bestimmten Lebensmitteln in Maßen erwünscht sind und zu deren charakteristischen Geschmack beitragen (z.B. Coffein in Tee oder Kaffee, Chinin in so genannten Bitter-Lemon-Getränken, Bitterstoffe wie Humolone oder iso-alpha-Säuren aus Hopfen in Bier), andererseits den Wert aber auch stark mindern können. Dazu gehören z.B. Flavonoidglycoside und Limonoide in Zitrussäften, der bittere Nachgeschmack vieler hochintensiver Süßstoffe wie Aspartam, Cyclamat, Acesulfam K, Rebaudiosid A, Glyccyrhizinsäure oder Saccharin sowie der unangenehme Geschmack, den hydrophobe Aminosäuren und Peptide in Käse verursachen können.

Bittergeschmack wird regelmäßig durch einzelne Stoffe verursacht, die an spezielle Bitterrezeptoren auf Geschmackszellen, die in den so genannten Geschmacksknospen auf der Zunge zu finden sind, binden und über neurochemische Kaskaden ein Signal an das Gehirn senden, dass eine Abwehrreaktion und einen negativen Geschmackseindruck verursacht (vgl. Meyerhof, Reviews of Physiology, Biochemistry and Pharmacology 2005, 154, 37-72**)**.

Daher ist es wünschenswert, bittermaskierende Stoffe zu finden, die unangenehme Geschmackseindrücke, insbesondere bittere, adstringierende und/oder metallische Geschmackseindrücke wirkungsvoll verändern, vermindern oder gar unterdrücken können, ohne dabei die Qualität eines entsprechenden Lebensmittels bzw. einer entsprechenden zum Verzehr geeigneten Zubereitung durch zusätzliche Prozessschritte zu beeinflussen. Dabei werden hier als bittermaskierende Stoffe Moleküle verstanden, die direkt auf physiologischer Ebene die Rezeption des Bittergeschmacks beeinflussen können; nicht gemeint sind z.B. komplexierende Stoffe wie Cyclodextrine oder Ionentauscherharze, die einfach nur die effektive Konzentration der Bitterstoffe senken sowie Stoffe, die einen angenehmen Geschmack, z.B. salzig, süß oder umami verursachen und dabei den bitteren Geschmack indirekt überdecken.

Üblicherweise werden bittermaskierende Stoffe mit folgenden Verfahren identifiziert:

- durch sensorische Methoden, indem man den Geschmack einer Mischung des Bitterstoffs mit dem Bitter-Antagonist gegen den Geschmack des Bitterstoffs alleine vergleicht;
- durch ein Screening in Gegenwart des Bitterstoffs mit/ohne Bitter-Antagonist mit Hilfe eines heterolog exprimierten Bitterrezeptors und ggfs. Helfermolekülen auf immortalisierten tierischen, bevorzugt menschlichen Zellen (vgl. z.B. WO 2004 029087 A1**,** WO 2008 057470 A1**);**
- durch ein Screening in Gegenwart des Bitterstoffs mit/ohne Bitter-Antagonist mit nativen humanen oder immortalisierten humanen Geschmackszellen; oder
- mit Hilfe von computergestützten Vorhersagen anhand von Pharmakophor-Modellen und/oder Homologie-Modellen der entsprechenden Rezeptoren.

Sensorisches Screening ist zeit- und personalaufwändig und kann nur mit toxikologisch unbedenklichen Substanzen durchgeführt werden. Bei den heterolog exprimierten Systemen ist die Korrelation zur sensorischen Wirklichkeit oft nur unzureichend abzubilden und zudem wird zur Vereinfachung nur ein Rezeptor exprimiert; da meist zur Bestimmung der Aktivierung ein Calcium-sensitiver Fluoreszensfarbstoff zugegeben wird, sind Störungen insbesondere bei höheren Testsubstanzkonzentrationen häufig. Primäre oder immortalisierte Geschmackszellen sind in der Regel nur schwer zu kultivieren und für eine Geschmacks- oder Bitterqualität stabil zu gewinnen; zudem ist die Rezeptorzusammensetzung nicht immer konstant. Die rechnerischen Methoden sind in der Regel zu unspezifisch, da für Geschmacksrezeptoren bisher keine für die Homologie-Berechnung wichtigen experimentellen 3-dimensionalen Strukturdaten beispielsweise als Röntgenkristallstruktur vorliegen.

Die komplexe Aufgabe der vorliegenden Erfindung war es daher, ein Screening-Verfahren zur Identifizierung speziell von Bitter-Antagonisten zu entwickeln, das gleichzeitig

(i) auf einer für das Hochdurchsatzscreening geeigneten menschlichen Zellkultur basiert,

(ii) dabei nicht auf heterolog exprimierte Bitterrezeptoren zurückgreift,

(iii) in der Regel nicht auf der direkten Messung der Änderung des Calciumspiegels in der Zelle beruht,

(iv) zu der menschlichen Geschmackswahrnehmung eine sehr hohe Korrelation zeigt, und sowohl

(v) Bitterstoffe als auch bittermodulierende Stoffe, d.h. bittermaskierende oder bitterverstärkende Stoffe identifiziert.

**Beschreibung der Erfindung**

Gegenstand der Erfindung ist ein in vitro-Verfahren zur Identifizierung von Bitterstoffen und bittermodulierenden Stoffen, bei dem man Testsubstanzen mit isolierten Magenzellen oder Magentumor-Zelllinien in Kontakt bringt und die dadurch hervorgerufene Änderung des intrazellularen pH-Wertes und/oder die zellulare Protonensekretion bestimmt.

Überraschend wurde festgestellt, dass insbesondere in kultivierten Magenzellen über die Änderung des interzellularen pH-Wertes bzw. die Protonensekretioneine sehr gute Korrelation zwischen sensorischen Eigenschaften und in vitro-Daten anhand bekannter Testsubstanzen (bittermaskierenden und nicht-wirksamen Testsubstanzen) liefern und daher das Testsystem hervorragend geeignet ist, eine Vielzahl von Testsubstanzen zum Zweck der Identifizierung von Bitter-Antagonisten für die oben genannten Bitterstoffe darstellt.

**Zellsysteme**

Als geeignete Zellsysteme haben sich isolierte Magenzellen und Magentumorzelllinien als besonders vorteilhaft erwiesen. Es hat sich gezeigt, dass insbesondere HGT-1-Zellen Gene von Bitterrezeptoren exprimieren. Von Vorteil ist insbesondere die gleichzeitige Ausprägung mehrerer Bitterrezeptoren, die sich für die anschließende sensorische Vergleichsmessung als besonders günstig herausgestellt hat. Neben den HGT-1-Zellen kommen auch alternative Zellsysteme in Betracht, beispielsweise isolierte Magenzellen der Provenienz Ratte *(Isolated rat gastric mucosal cells)[1]*, Hase *(isolated gastric glands from white rabbits)[2]*, Maus[3] oder Meerschweinchen[4]. Ebenfalls geeignet sind Magentumor Zell-Linien, wie etwa MKN-45, AGS, KATO3, SNU16, oder SNU5.

[1]Dixit, C.; Dikshit, M., A flowcytometric method for evaluation of acid secretion from isolated rat gastric mucosal cells. J Pharmacol Toxicol Methods 2001, 45, 47-53.

[2]Matsuno, K.; Tomita, K.; Okabe, S., Wine stimulates gastric acid secretion in isolated rabbit gastric glands via two different pathways. Aliment Pharmacol Ther 2002, 16 Suppl 2, 107-14.

[3]Vila-Petroff, M.; Mundiha-Weilenmann, C.; Lezcano, N.; Snabaitis, A. K.; Huergo, M. A.; Valverde, C. A.; Avkiran, M.; Mattiazzi, A., Ca(2+)/calmodulin-dependent protein kinase II contributes to intracellular pH recovery from acidosis via Na(+)/H(+) exchanger activation. J Mol Cell Cardiol 2010, 49, 106-12.

[4]Swietach, P.; Rossini, A.; Spitzer, K. W.; Vaughan-Jones, R. D., H+ ion activation and inactivation of the ventricular gap junction: a basis for spatial regulation of intracellular pH. Circ Res 2007, 100, 1045-54.

Bisher wurden HGT-1-Zellen als Messsystem für die Identifizierung und Charakterisierung von Substanzen genutzt, die die Magensäuresekretion beeinflussen können, z.B. bestimmte nur sauer schmeckende Fruchtsäuren (vgl. Liszt, et al., J. Agric. Food Chem. 60, (28), 7022-7030 (2012)**.**

Es ist auch bekannt, dass ausgewählte bitter-schmeckende Stoffe wie z.B. Hopfeninhaltsstoffe (vgl. Walker, et al., J. Agric. Food Chem. 60, (6), 1405-1412 (2012**)** oder aus Kaffee (vgl. Rubach et al., J. Agric. Food Chem. 58, 4153-61 (2010**)** die Sekretion beeinflussen können, ein genereller Zusammenhang zwischen der sensorischen Eigenschaft "bitter" und der physiologischen Reaktion "Säuresekretion" wurde bisher aber kein Zusammenhang gesehen. Daher war es überraschend, dass das die HGT-1-Zellen auch als Messsystem für die Identifizierung und Charakterisierung von bitteren Agonisten geeignet ist, was einen weiteren Aspekt der Erfindung darstellt.

Zudem wurde als Target für die Reduktion der Sekretion auf der Zelloberfläche der HGT-1 (human gastric tumor cell line) bisher nur der Somatostatin-Rezeptor (SSTR2), der Histaminrezeptor (HRH2) sowie der Acetylcholinrezeptor (CHRM3) beschrieben. Bisher sind diese Zellen aber immer nur als Einzelstoffe in Kontakt mit potentiellen Regulatoren der Protonensekretion gebracht worden, die geneinsame Verabreichung von bitteren Agonisten und potentiellen Bitter-Modulatoren, insbesondere Bitter-Maskierern ist bisher nicht beschrieben worden.

Zwar sind in einer Vielzahl von nicht-oralen Geweben Geschmacksrezeptoren beschrieben worden (vgl. Behrens, et al., Physiology & Behavior 105, (1), 4-13 (2011**)** in keinem Fall mit Ausnahme der Mundhöhle ist aber bisher ein Nachweis gelungen, dass deren Aktivierung zu einem bewusst wahrnehmbaren sensorischen Ereignis oder Geschmackserlebnis führt.

Daher war es überraschend und für den Fachmann nicht vorhersehbar, dass die isolierte Magenzellen oder Magentumorzelllinien im Allgemeinen und beispielsweise HGT-1-Zellen im Besonderen mit dem erfindungsgemäßen Verfahren zur Identifizierung von geschmacksmodulierenden, insbesondere bitter-modulierenden Stoffen geeignet sein könnten.

Mit Hilfe des erfindungsgemäßen Verfahrens lassen sich vorzugsweise bitter-maskierende Stoffe identifizieren. Vereinfacht ausgedrückt, vergleicht man die Entwicklung des pH-Wertes, d.h. die Freisetzung von Protonen in zwei gleichartigen Zellkulturen, wobei man der einen Probe nur den bekannten Bitterstoff zusetzt und der anderen den bekannten Bitterstoff zusammen mit der oder den Testsubstanz(en). Die freigesetzten Protonenmengen werden gemessen und die Differenz der T/C-Werte (treatment over control) gebildet. Gilt die Gleichung

$$\text{T/C (bekannter Bitterstoff)} - \text{T/C (bekannter Bitterstoff + Testsubstanz)} > 0$$

d.h. ist die Differenz positiv (Wert der Kontrolle = 0), bedeutet dies, dass in der Probe, die die Testsubstanz enthalten hat, weniger Protonen freigesetzt worden sind. Ist die Abweichung von 0 signifikant, dann handelt es sich um einen potentiellen Bittermaskierer, dessen Eignung in weiteren sensorischen Untersuchungen evaluiert werden muss. Signifikant ist die Abweichung dann, wenn die relative Differenz zwischen den beiden Werten mindestens 10 %, vorzugsweise mindestens 20 % und insbesondere mindestens 30 % des größeren Wertes ausmacht.

Das Verfahren lässt sich umgekehrt ebenfalls nutzen, um bitterverstärkende Stoffe zu identifizieren. Hier muss dann die Gleichung

$$\text{T/C (bekannter Bitterstoff)} - \text{T/C (bekannter Bitterstoff + Testsubstanz)} < 0$$

lauten, d.h. ist die Differenz negativ (Wert der Kontrolle = 0) bedeutet das, dass in der Probe, die die Testsubstanz enthalten hat, mehr Protonen freigesetzt worden sind. Auch hier ist die Abweichung dann signifikant, wenn die relative Differenz zwischen den beiden Werten mindestens 10 %, vorzugsweise mindestens 20 % und insbesondere mindestens 30 % des größeren Wertes ausmacht.

Das Verfahren eignet sich ebenfalls um potentielle Bitterstoffe selbst zu identifizieren. In diesem Fall gilt dann

$$\text{T/C (Kontrolle)} - \text{T/C (Testsubstanz)} < 0$$

wobei T/C für den Wert der Protonensekretion in Prozent steht und außerdem die Bedingung besteht, dass die relative Differenz zwischen beiden Werten [der Wert der neutralen Kontrolle (Lösungsmittel und Puffer ohne Testsubstanz) und der Wert der Testsubstanz] mindestens 10 % des größeren Wertes ausmacht. In diesem Spezialfall wird also die Testsubstanz ohne Agonisten zugesetzt und untersucht, ob es zu einer signifikanten Verstärkung der Protonensekretion kommt.

Ein weiterer Gegenstand der Erfindung betrifft daher Verfahren zur Identifizierung von bittermodulierenden Stoffen der oben beschriebenen Art, bei dem man

(a) eine einheitliche Kultur eines Zellsystems ausgewählt aus der Gruppe, die gebildet wird von isolierten Magenzellen oder Magentumor-Zelllinien, zur Verfügung stellt und diese in zwei Proben teilt,

(b) zur ersten Probe einen bekannten Bitterstoff (Bitteragonist) gibt,

(c) zur zweiten Probe den gleichen Bitterstoff aus (b) und mindestens eine Testsubstanz gibt, wobei diese bittermodulierende (d.h. bitter-verstärkende oder bitter-maskierende) Stoffe darstellen können,

(d) die Änderung des intrazellulären pH-Wertes und/oder der Protonensekretion der Zellen über die Experimentalzeit misst,

(e) nach Ablauf der Experimentalzeit die Differenz zwischen der Änderung des intrazellulären pH-Wertes und/oder der Protonensekretion der Zellen der ersten und der zweiten Probe bildet

(f) die Testsubstanzen auswählt, bei denen die Differenz entweder positiv oder negativ ist und die relative Differenz zwischen den beiden Werten mindestens 10 % des größeren Wertes ausmacht,

(g) und gegebenenfalls die so ermittelten Testsubstanzen anschließend einer sensorischen Beurteilung unterwirft.

Zusätzlich wird ein Verfahren zur Identifizierung von Bitterstoffen der oben beschriebenen Art beansprucht, bei dem man

(a) eine einheitliche Kultur eines Zellsystems ausgewählt aus der Gruppe, die gebildet wird von isolierten Magenzellen oder Magentumor-Zelllinien, zur Verfügung stellt und diese in zwei Proben teilt,

(b) zur ersten Probe eine Kontrolllösung ohne Testsubstanz,

(c) zur zweiten Probe eine Testsubstanz gibt,

(d) die Änderung des intrazellulären pH-Wertes und/oder der Protonensekretion der Zellen über die Experimentalzeit misst,

(e) nach Ablauf der Experimentalzeit die Differenz zwischen der Änderung des intrazellulären pH-Wertes und/oder der Protonensekretion der Zellen der ersten und der zweiten Probe bildet

(f) die Testsubstanzen auswählt, bei denen die Differenz negativ ist und die relative Differenz zwischen den beiden Werten mindestens 10 % des größeren Wertes ausmacht,

(g) und gegebenenfalls die so ermittelten Testsubstanzen anschließend einer sensorischen Beurteilung zur Bestimmung der Bitterwirkung unterwirft.

**Bekannte Bitterstoffe (Bitter-Agonisten)**

Unter den bekannten Bitterstoffen (Bitter-Agonisten), die im Sinne des erfindungsgemäßen Verfahrens den HGT-1 Zellkulturen zugesetzt werden, versteht man hier Stoffe, die alleine in ausreichender Menge in wässrigen Lösungssystemen am Menschen bekanntermaßen einen bitteren Geschmack auslösen können und einen oder mehreren der bekannten 25 menschlichen Bitter-Rezeptoren, insbesondere Rezeptoren vom Typ TAS2R1, TAS2R3, TAS2R7, TAS2R16, TAS2R30, TAS2R38, TAS2R40 und TAS2R50 aktivieren können
Vorzugsweise sind die Bitter-Agonisten ausgewählt aus der Gruppe, die gebildet wird von:

- Xanthinalkaloiden, z.B. Coffein, Theobromin, Theophyllin;

- Alkaloiden, z.B. Chinin, Brucin, Strychnin, Nicotin;

- phenolischen Glycosiden, z.B. Salicin, Sinigrin, Arbutin;

- Flavanoidglycosiden, z.B. Neohesperidin, Eriocitron, Neoeriocitrin, Narirutin, Naringin;

- Chalconen oder Chalconglycosiden, Dihydrochalconglycosiden, z.B. Phloridzin, Trilobatin);

- hydrolisierbaren Tanninen, z.B. Gallus- oder Elagsäureester von Kohlenhydraten, z.B. Pentagalloylglucose;

- nichthydrolisierbaren Tanninen, z.B. gegebenenfalls galloylierte Catechine oder Epicatechine und deren Oligomeren, z.B. Proanthyocyanidine oder Procyanidine, Thearubigenin), Flavone und deren Glycoside (z.B. Quercetin, Quercitrin, Rutin, Taxifolin, Myricetin, Myrictrin;

- anderen Polyphenolen, z.B. γ-Oryzanol, Kaffeesäureester;

- bitteren Isothiocyanaten oder abgeleiteten Stoffen wie Thiocarbamate, Thiourethane, Glucosinolate, Goitrin oder Propylthiouracil (PROP) oder Phenylthiocarbamat (PTC),

- terpenoiden Bitterstoffen, z.B. Menthol, Limonoide wie Limonin oder Nomilin aus Zitrusfrüchten, Lupolone und Humolone aus Hopfen sowie daraus abgeleitete iso-alpha-Säuren, Iridoide, Secoiridoide), Absinthin aus Wermut, Amarogentin aus Enzian;

- pharmazeutischen Wirkstoffen, z.B. Fluorchinolon-Antibiotika, Paracetamol, Aspirin, β-Lactam-Antibiotika, Ambroxol, Propylthiouracil [PROP], Omeprazol, Guaifenesin, Chloroquin;

- Denatoniumbenzoat oder anderen Denatoniumsalzen;

- Sucraloseoctaacetat;

- Harnstoff;

- Aminosäuren, z.B. Leucin, Isoleucin, Valin, Tryptophan, Prolin, Histidin, Tyrosin, Lysin oder Phenylalanin;

- Peptiden, insbesondere Peptide mit einer Aminosäure aus der Gruppe Leucin, Isoleucin, Valin, Tryptophan, Prolin oder Phenylalanin am N- oder C-Terminus

sowie deren Gemische.

Insbesondere bevorzugt sind hierbei Stoffe, die alleine in ausreichender Menge in wässrigen Lösungssystemen am Menschen einen bitteren Geschmack auslösen können und bevorzugt mindestens einen oder mehreren der Bitter-Rezeptor-Typen aktivieren können und die ausgewählt sind aus der Gruppe Coffein, Theobromin, Theophyllin, Salicin, Sinigrin, Arbutin, Chinin, Menthol, ggfs. galloylierte Catechine oder Epicatechine wie Epigallocatechin, Epigallocatechingallat, Epicatechingallat, Amarogentin, Limonoide wie Limonin oder Nomilin aus Zitrusfrüchten, Lupolone und Humolone aus Hopfen sowie daraus abgeleitete iso-alpha-Säuren, Aminosäuren (z.B. Leucin, Isoleucin, Valin, Tryptophan, Prolin, Histidin, Tyrosin, Lysin oder Phenylalanin), und Peptide (insbesondere Peptide mit einer Aminosäure aus der Gruppe Leucin, Isoleucin, Valin, Tryptophan, Prolin oder Phenylalanin am N- oder C-Terminus).

Mit dem oben angegebenen Verfahren können natürlich auch bisher nicht als bitterschmeckende Stoffe identifiziert werden.

## Testsubstanzen

Nachdem es Aufgabe des Verfahrens ist, Kandidaten aufzufinden, die bittermodulierende Eigenschaften aufweisen, kann an dieser Stelle keine vollständige Liste von Stoffen eingefügt werden, die über diese Eigenschaften verfügen, weil dies ja die Erfindung vorwegnehmen würde. Allerdings ist es möglich, Klassen von Stoffen anzugeben, innerhalb derer vorzugsweise nach geeigneten Kandidaten gesucht werden sollte. Für die bittermaskierenden Stoffe sind vor allem die Gruppen der Hydroxyflavone, der Hydroxybenzoesäureamide, der Hydroxydeoxybenzoine, der Hydroxyphenylalkandione, der 4-Hydroxydihydrochalcone sowie der Vanillyllignane zu nennen.

## Messverfahren

Die Durchführung des erfindungsgemäßen Verfahrens wird im Folgenden experimentellen Teil detailliert beschrieben, so dass an dieser Stelle nur auf einige grundsätzliche Aspekte eingegangen werden soll.

Die Messung des intrazellularen pH-Wertes erfolgt spektrometisch, vorzugsweise unter Einsatz eines Fluoreszenzfarbstoffes, bevorzugt eines Fluoreszenzfarbstoffes, der zur Bestimmung des intrazellulären pH-Wertes zwischen pH 6 und pH 8 geeignet ist, wie beispielsweise 2',7'-Bis-(2-carboxypropyl)-5-(and-6-)-carboxyfluorescein (BCECF) und seine Ester und/oder seine Salze, 8-Hydroxypyren-1,3,6-trisulfonsäure (HPTS) und seine Salze und/oder Ester, Carboxyfluorescein und seine Ester und/oder seine Salze, 1,5-Carboxy-seminaphtorhodafluor (SNARF) und seine Ester und/oder Salze, oder weitere, in Chem Rev. 2010 110(5):2709-2728 beschriebene Fluoreszenzfarbstoffe; insbesondere aber 1,5-Carboxyseminaphtorhodafluoracetoxymethylester (SNARF-1-AM). Als alternativer Farbstoff kommt auch Acridine Orange in Betracht sowie die Messung des $C^{14}$ markierten Aminopyrin, jene Methode die als Aminopyrine uptake[5] bekannt ist.

[5]Ding, X.; Deng, H.; Wang, D.; Zhou, J.; Huang, Y.; Zhao, X.; Yu, X.; Wang, M.; Wang, F.; Ward, T.; Aikhionbare, F.; Yao, X., Phospho-regulated ACAP4-Ezrin interaction is essential for histamine-stimulated parietal cell secretion. J Biol Chem 2010, 285, 18769-80.

Das erfindungsgemäße Verfahren wird an Hand der Änderung des pH-Wertes in den Zellkulturen (vorzgsweise vom Typ HGT-1) mit und ohne Testsubstanz durchgeführt. Damit eine pH-Wert-Änderung beobachtet werden kann, ist es empfehlenswert, eine Mindestexperimentalzeit einzuhalten, also den pH-Wert erst nach Erreichen eines Gleichgewichtszustandes zu bestimmen. Für die Stimulierung der Zellen ist in der Regel eine Zeitspanne von 10 min ausreichend, so dass die Experimentalzeit etwa 1 bis etwa 30 min, vorzugsweise etwa 5 bis etwa 20 min und insbesondere etwa 8 bis 14 min betragen sollte.

Die Zellkulturen werden in konventioneller Weise zusammen mit dem Farbstoff inkubiert und dann geteilt, wobei der einen Hälfte der bekannte Bitterstoff und der anderen die Mischung aus demselben Bitterstoff und einer oder mehrerer Testsubstanzen zugesetzt wird. Typische Mengen an bekanntem Bitter-Agonist liegen bei 50 bis 150 μl, die Testsub-stanzen werden in der Regel in Konzentrationen von etwa 0,1 bis 3.000 μM eingesetzt. Es hat sich als vorteilhaft erwiesen, die Testsubstanzen in unterschiedlichen Mengen einzusetzen, beispielsweise in Konzentrationen von 0,1, 1, 10, 100 und 1.000 μM, um auszuschließen, dass ein geeigneter Kandidat unerkannt bleibt, weil man ihn beispielsweise zu gering dosiert hat.

Aus dem Verhältnis der Wellenlängen bei Anregung und Emission des Fluoreszenzfarbstoffs kann eine Kalibrierkurve erstellt werden, mit deren Hilfe sich der pH-Wert in den Proben einfach bestimmen lässt. Für die Kalibrierkurve werden die Zellen vorzugsweise mit einem Kaliumpuffer unterschiedlicher pH-Werte von 7,2 - 8,2 pH und 2 $\mu$M Nigericin behandelt. Nigericin equilibriert den intrazellulären und extrazellulären pH-Wert, sodass der intrazelluläre pH-Wert definiert werden kann. Die intrazelluläre H$^+$- Konzentration wird aus dem intrazellulären pH-Wert ermittelt. Die reduzierte Menge an intrazellulären Protonen bzw. die Menge an freigesetzten Protonen wird kalkuliert durch log2 Transformation des Verhältnisses zwischen behandelten Zellen und unbehandelten Zellen (Kontrolle). Die so erhaltenen Ergebnisse werden dann als prozentuale Veränderung im Vergleich zu nicht-behandelten Kontrollzellen angegeben (vgl. Malte Rubach, R. L., Elisabeth Seebach, Mark M. Somoza, Thomas Hofmann; Somoza, a. V., Mol Nutr Food Res. in press, 2011; Rubach, M.; Lang, R.; Hofmann, T.; Somoza, V., Ann N Y Acad Sci 2008, 1126, 310-4; Rubach, M.; Lang, R.; Skupin, C.; Hofmann, T.; Somoza, V., J Agric Food Chem 2010, 58, 4153-61; Weiss, C.; Rubach, M.; Lang, R.; Seebach, E.; Blumberg, S.; Frank, O.; Hofmann, T.; Somoza, V., J Agric Food Chem 2010, 58, 1976-85; Liszt, K. I.; Walker, J.; Somoza, V., J Agric Food Chem 2012; Walker, J.; Hell, J.; Liszt, K. I.; Dresel, M.; Pignitter, M.; Hofmann, T.; Somoza, V., J Agric Food Chem 2012, 60, 1405-12).

## Industrielle Anwendbarkeit

Das erfindungsgemäße Verfahren ist geeignet, unterschiedliche geschmacksmodulierende bzw. bittermodulierende oder auch bitter schmeckende Stoffe aufzufinden, nämlich zum einen Bittermaskierer und zum anderen Bitterverstärker. Der Anwendungsbereich umfasst sowohl den Bereich der Nahrungsmittel als auch den der Pharmazie.

## Nahrungsmittel

Bei den Nahrungsmitteln, denen die nach dem erfindungsgemäßen Verfahren identifizierten bitter schmeckenden, bittermaskierenden oder bitterverstärkenden Stoffe, bevorzugt aber bittermaskierenden Stoffe zugesetzt werden können, handelt es sich um Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchproteinhaltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

## Pharmazeutische Zubereitungen

Zu den pharmazeutischen Zubereitungen, denen man die bitter schmeckenden, bittermaskierenden oder bitterverstärkenden Stoffe, bevorzugt aber bittermaskierenden Stoffe ebenfalls zusetzen kann, zählen beispielsweise steroidale entzündungshemmende Substanzen vom Kortikosteroiden-Typ wie beispielsweise Hydrocortison, Hydrocortison-Derivate wie Hydrocortison-17-butyrat, Dexamethason, Dexamethasonphosphat, Methylprednisolon oder Cortison.
Vorteilhafte nichtsteroidale pharmazeutische Wirkstoffe, denen die Teststoffe ebenfalls zugesetzt werden können, sind beispielsweise Entzündungshemmer wie Oxicame wie Piroxicam oder Tenoxicam; Salicylate wie Aspirin® (Acetylsalicylsäure), Disalcid, Solprin oder Fendosal; Essigsäure-Derivate wie Diclofenac, Fenclofenac, Indomethacin, Sulindac, Tolmetin, oder Clindanac; Fenamate wie Mefenamic, Meclofenamic, Flufenamic oder Niflumic; Propionsäure-Derivate wie Ibuprofen, Naproxen, Flurbiprofen, Benoxaprofen oder Pyrazole wie Phenylbutazon, Oxyphenylbutazon, Febrazon oder Azapropazon.

Besonders bevorzugte pharmazeutische Zubereitungen sind nicht verschreibungspflichtige Produkte und frei verkäufliche Arzneimittel, sogenannte OTC ("over the counter") - Präparate, enthaltend Wirkstoffe wie Paracetamol, Acetylsalicylsäure oder Ibuprofen, Vitamine (beispielsweise Vitamin H, Vitamine aus der B-Reihe wie Vitamin B1, B2, B6, B12, Niacin, Panthotensäure, vorzugsweise in Form von (Brause)Tabletten oder Kapseln), Mineralien (vorzugsweise in Form von (Brause)Tabletten oder Kapseln) wie Eisensalze, Zinksalze, Selensalze, Produkte enthaltend Wirkstoffe oder Extrakte von Spitzwegerich (beispielsweise in Hustensirup) oder Johanniskraut.

Es versteht sich, dass diese Aufzählung nur illustrierenden und keinen limitierenden Charakter besitzt.

**Beispiele**

**Zellmodell**

Als Zellmodell werden humane Parietalzellen, auch als human gastric tumor cell line (HGT-1) bekannt, verwendet. Diese wurden von Dr. C. Laboisse (Laboratory of Pathological Anatomy, Nantes, France) zur Verfügung gestellt. Die Zellen werden unter den Standardbedingungen 37 °C, 5 % $CO_2$ in DMEM mit 4 g/L Glucose, 10 % FBS, 2 % L-Glutamin und 1 % Penicillin/Streptomycin kultiviert. Für die Reverse Transkription- quantitative PCR (RT-qPCR) und die Messung der intrazellulären Protonenkonzentration werden die Zellen mit Trypsin/EDTA geerntet, die Viabilität mittels Trypanblaufärbung bestimmt und die Zellen in definierter Anzahl in einer 35 mm Zellkulturschale beziehungsweise in schwarzen 96-well Platten ausgesät.

**Beispiel 1**

Nachweis der Expression von Bitter-Rezeptoren in HGT-1 Zellen mittels RT-qPCR

Die RNA der HGT-1 Zellen werden mit dem RNeasy Mini Kit (Qiagen) isoliert, spektrometrisch überprüft und mit dem High Capacity cDNA Reverse Transcription Kit (Applied Biosystems) revers transkribiert. Die Primer werden mit Hilfe des Primer designing tool von NCBI (unter Verwendung von Primer 3 und Blast) konstruiert. Die qPCR wird mit dem StepOne plus Gerät (Applied Biosystems) und dem Fast SYBR green Mastermix (Applied Biosystems) durchgeführt. Die qPCR verläuft nach folgendem Schema: Aktivierung der Polymerase bei 95°C für 20 s, Zyklus: 95°C für 3 s (Denaturierung), 60°C für 30 s (Annealing) und 72°C für 15 s (Elongation und Fluoreszenzmessung). Die PCR-Produkte werden mittels Schmelzkurve und Bestimmung der Ampliconlänge am Agarosegel überprüft. Die Expression der Bitter-Rezeptoren wird dargestellt als Differenz der Ct- Werte der Bitter-Rezeptoren zu den Ct-Werten der endogenen Kontrolle PPIA (Peptidylprolyl Isomerase A).

In der folgenden **Tabelle 1** ist die mRNA Expression der Bitter-Rezeptoren in HGT-1 Zellen wiedergegeben. Die Ergebnisse sind dargestellt als Mittelwert und Standardabweichung, n=1 (n, Anzahl der biologischen Replikate), tr=3 (tr, Anzahl der technischen Replikate).

Tabelle 1

| mRNA Expression | | |
|---|---|---|
| **Rezeptor** | **$\Delta$CT Zu PPIA** | **Standardabweichung** |
| AS2R1 | 14.41 | 0.12 |
| TAS2R3 | 7.02 | 0.05 |
| TAS2R7 | 7.89 | 0.15 |
| TAS2R16 | 6.96 | 0.09 |
| TAS2R30 | 7.81 | 0.04 |
| TAS2R38 | 9.85 | 0.20 |
| TAS2R40 | 9.67 | 0.12 |
| TAS2R50 | 5.67 | 0.07 |

**Beispiel 2**

Identifizierung von potentiellen Bitterstoffen durch induzierte Protonensekretion in HGT-1 Zellen

Zur Messung des intrazellularen pH-Wertes wird der Farbstoff 1,5 Carboxy-Seminaphtorhodafluor Acetoxymethylester (SNARF-1-AM) verwendet. Die Zellen in den 96-well Platten werden einmal mit Krebs-Hepes-Puffer (KRHP) gewaschen und mit dem Farbstoff in der Konzentration von 3 $\mu$M gelöst in KRHP für 30 min bei 37 °C und 5 % $CO_2$ inkubiert. Danach werden die Zellen zwei Mal mit KRHP gewaschen und die Bitter-Agonisten, beispielsweise Coffein, Theobromin und Naringin in unterschiedlichen Konzentrationen in phenolrotfreiem DMEM im Volumen von 100 $\mu$L aufgetragen. Der Fluoreszenzfarbstoff wird bei der Wellenlänge von 488 nm angeregt und die Emission bei 580 nm und 640 nm gemessen. Das Verhältnis der Fluoreszenzwerte von 580 nm zu 640 nm wird aufgetragen im Vergleich zur Kalibrierkurve, woraus der pH-Wert ermittelt werden kann. Für die Kalibrierkurve werden die Zellen mit einem Kaliumpuffer unterschiedlicher pH-Werte von 7,2 - 8,2 pH und 2 $\mu$M Nigericin behandelt. Nigericin equilibriert den intrazellulären und extrazellulären pH-Wert, sodass der intrazelluläre pH-Wert definiert werden kann. Die intrazelluläre $H^+$- Konzentration wird aus dem intrazellulären pH-Wert ermittelt. Die reduzierte Menge an intrazellulären Protonen bzw. die Menge an freigesetzten Protonen wird kalkuliert durch log2 Transformation des Verhältnisses zwischen behandelten Zellen und unbehandelten Zellen (Kontrolle). Die hier dargestellten Ergebnisse werden als prozentuale Veränderung im Vergleich zu nicht-behandelten Kontrollzellen angegeben.

In der folgenden **Tabelle 2A** ist die Protonensekretion in HGT-1 Zellen nach 10 minütiger Stimulierung durch Histamin (1mM, Positivkontrolle) oder Coffein in unterschiedlichen Konzentrationen wiedergegeben. Die Daten sind dargestellt als Mittelwert und mittlere Standardabweichug, n=5, tr=6; Statistik: one-way Anova mit post-hoc Test nach Dunn's. Signifikante Unterschiede ($p < 0,05$) werden durch Buchstaben angegeben.

**Tabelle 2A**

| Protonensekretion durch die Kontrollsubstanz Histamin oder den Bitterstoff Coffein | | |
|---|---|---|
| **Testsubstanz** | **T/C [%]** | **SEM** |
| Kontrolle | 1.09 | 5.14 a |
| Histamin | 36.66 | 4.93 b |
| Coffein 0,3 $\mu$M | 25.49 | 5.02 a,b |
| Coffein 3 $\mu$M | 14.74 | 3.60 a,b |
| Coffein 30 $\mu$M | 23.43 | 3.91 a,b |
| Coffein 300 $\mu$M | 17.07 | 3.54 a,b |
| Coffein 3000 $\mu$M | 40.97 | 2.72 b |

In **Tabelle 2B** ist die Protonensekretion in HGT-1 Zellen nach 10 minütiger Stimulierung durch Histamin (1mM, Positivkontrolle) oder Theobromin in unterschiedlichen Konzentrationen angegeben. Die Daten sind dargestellt als Mittelwert und mittlere Standardabweichug, n=4, tr=6. Statistik: one-way Anova mit post-hoc Test nach Dunn's. Signifikante Unterschiede ($p < 0,05$) werden durch Buchstaben angegeben.

**Tabelle 2B**

| Protonensekretion durch die Kontrollsubstanz Histamin oder den Bitterstoff Theobromin | | |
|---|---|---|
| **Testsubstanz** | **T/C [%]** | **SEM** |
| Kontrolle | -3.25 | -5.64 a |
| Histamin | 45.70 | -5.87 b |
| Theobromin 0,03 $\mu$M | 3.71 | -7.99 c,a |
| Theobromin 0,3 $\mu$M | 17.71 | -9.06 c,a |
| Theobromin 3 $\mu$M | 43.16 | -11.6 b,c |
| Theobromin 30 $\mu$M | 44.88 | -7.4 b |
| Theobromin 300 $\mu$M | 57.2 | -11.18 b |

**Beispiel 3**

Identifizierung von Bitter-Maskierern durch Messung der Abnahme der durch Bitterstoffe induzierte Protonensekretion in HGT-1 Zellen

Zur Messung des intrazellularen pH-Wertes wird der Farbstoff 1,5 Carboxy-Seminaphtorhodafluor Acetoxymethylester (SNARF-1-AM) verwendet. Die Zellen in den 96-well Platten werden einmal mit Krebs-Hepes-Puffer (KRHP) gewaschen und mit dem Farbstoff in der Konzentration von 3 $\mu$M gelöst in KRHP für 30 min bei 37 °C und 5 % $CO_2$ inkubiert. Danach werden die Zellen zwei Mal mit KRHP gewaschen und mit Bitterstoff, beispielsweise 3 mM Coffein beziehungsweise 0,3 mM Theobromin in Kombination mit Bittermaskierern, beispielsweise Homoeriodictyol (HED) oder Eriodictyol oder Matairesinol oder Lariciresinol in unterschiedlichen Konzentrationen in phenolrotfreiem DMEM im Volumen von 100 $\mu$L aufgetragen. Homoeriodictyol wird in doppelt destilliertem Wasser gelöst während Eriodictyol, Matairesinol und Lariciresinol in EtOH gelöst werden. Die finale Konzentration an Lösungsmittel, die den Zellen zugeführt wird, beträgt maximal 1 %. Der Fluoreszenzfarbstoff wird bei der Wellenlänge von 488 nm angeregt und die Emission bei 580 nm und 640 nm gemessen. Das Verhältnis der Fluoreszenzwerte von 580 nm zu 640 nm wird aufgetragen im Vergleich zur Kalibrierkurve, woraus der pH-Wert ermittelt werden kann. Für die Kalibrierkurve werden die Zellen mit einem Kaliumpuffer unterschiedlicher pH-Werte von 7,2 - 8,2 pH und 2 $\mu$M Nigericin behandelt. Nigericin equilibriert den intrazellulären und extrazellulären pH-Wert, sodass der intrazelluläre pH-Wert definiert werden kann. Die intrazelluläre H$^+$- Konzentration wird aus dem intrazellulären pH-Wert ermittelt. Die reduzierte Menge an intrazellulären Protonen bzw. die Menge an freigesetzten Protonen wird kalkuliert durch log2 Transformation des Verhältnisses zwischen behandelten Zellen und unbehandelten Zellen (Kontrolle). Die hier dargestellten Ergebnisse werden als prozentuale Veränderung im Vergleich zu nicht-behandelten Kontrollzellen angegeben. Die Anzahl der angegebenen Replikate beziehen sich auf technische Replikate (tr) oder auf die Anzahl an Gesamtreplikaten (n), die sich aus der Anzahl der technischen Replikate multipliziert mit der Anzahl an biologischen Replikaten ergibt.

In der folgenden **Tabelle 3A** ist die prozentuale Steigerung der Protonensekretion in HGT-1 Zellen im Vergleich zu unbehandelten Kontrollen nach 10 minütiger Stimulierung durch 3 mM Coffein oder 3 mM Coffein in Kombination mit Homoeriodictyol (HED) in unterschiedlichen Konzentrationen angegeben. Die Daten sind dargestellt als Mittelwert und mittlere Standardabweichung, n=4, tr=6. Statistik: one-way Anova mit post-hoc Test nach Dunn's. Signifikante Unterschiede (p < 0,05) werden durch Buchstaben angegeben.

**Tabelle 3A**

| Senkung der durch den Bitterstoff Coffein ausgelösten Sekretion durch den Bittermaskierer Homoeriodictyol. | | |
|---|---|---|
| **Testsubstanz** | **T/C [%]** | **SEM** |
| 3 mM Coffein | 54.79 | 5.61 a |
| 3 mM Coffein + 0.03 mM HED | 37.99 | 6.70 a |
| 3 mM Coffein + 0.3 mM HED | 20.18 | 5.99 b |

In der folgenden Tabelle 3B ist die prozentuale Steigerung der Protonensekretion in HGT-1 Zellen im Vergleich zu unbehandelten Kontrollen nach 10 minütiger Stimulierung durch 3 mM Coffein oder 3 mM Coffein in Kombination mit Eriodictyol in unterschiedlichen Konzentrationen dargestellt. Die Daten sind dargestellt als Mittelwert und mittlere Standardabweichung, n=4, tr=6. Statistik: one-way Anova mit post-hoc Test nach Dunn's. Signifikante Unterschiede (p < 0,05) werden durch Buchstaben angegeben.

**Tabelle 3B**

| Senkung der durch den Bitterstoff Coffein ausgelösten Sekretion durch den Bittermaskierer Eriodictyol. | | |
|---|---|---|
| **Testsubstanz** | **T/C [%]** | **SEM** |
| 3 mM Coffein | 54.05 | 4.37 a |
| 3 mM Coffein + 0.03 mM Eriodictyol | 29.69 | 8.82 a |
| 3 mM Coffein + 0.3 mM Eriodictyol | -33.96 | 7.55 b |

In der folgenden Tabelle 3C ist die prozentuale Steigerung der Protonensekretion in HGT-1 Zellen im Vergleich zu unbehandelten Kontrollen nach 10 minütiger Stimulierung durch 3 mM Coffein oder 3 mM Coffein in Kombination mit Matairesinol in unterschiedlichen Konzentrationen dargestellt. Die Daten sind dargestellt als Mittelwert und mittlere Standardabweichung, n=4, tr=6. Statistik: one-way Anova mit post-hoc Test nach Dunn's. Signifikante Unterschiede (p < 0,05) werden durch Buchstaben angegeben.

**Tabelle 3C**

| Senkung der durch den Bitterstoff Coffein ausgelösten Sekretion durch den Bittermaskierer Matairesinol. | | |
|---|---|---|
| **Testsubstanz** | **T/C [%]** | **SEM** |
| 3 mM Coffein | 38.75 | 2.63 a |
| 3 mM Coffein + 0.03 mM Matairesinol | 14.39 | 3.55 b |
| 3 mM Coffein + 0.3 mM Matairesinol | -20.43 | 4.82 c |

In der folgenden **Tabelle 3D** ist die prozentuale Steigerung der Protonensekretion in HGT-1 Zellen im Vergleich zu unbehandelten Kontrollen nach 10 minütiger Stimulierung durch 3 mM Coffein oder 3 mM Coffein in Kombination mit Lariciresinol in unterschiedlichen Konzentrationen wiedergegeben. Die Daten sind dargestellt als Mittelwert und mittlere Standardabweichung, n=5, tr=6. Statistik: one-way Anova mit post-hoc Test nach Dunn's. Signifikante Unterschiede (p < 0,05) werden durch Buchstaben angegeben.

**Tabelle 3D**

| Senkung der durch den Bitterstoff Coffein ausgelösten Sekretion durch den Bittermaskierer Lariciresinol. | | |
|---|---|---|
| **Testsubtanz** | **T/C [%]** | **SEM** |
| 3 mM Coffein | 52.41 | 4.16 a |
| 3 mM Coffein + 0.03 mM Lariciresinol | 46.40 | 3.98 a |
| 3 mM Coffein + 0.3 mM Lariciresinol | 21.57 | 3.78 b |

In der folgenden **Tabelle 3E** ist die prozentuale Steigerung der Protonensekretion in HGT-1 Zellen im Vergleich zu unbehandelten Kontrollen nach 10 minütiger Stimulierung durch 0.3 mM Theobromin oder 0.3 mM Theobromin in Kombination mit Homoeriodictyol (HED) in unterschiedlichen Konzentrationen wiedergegeben. Die Daten sind dargestellt als Mittelwert und mittlere Standardabweichung, n=3, tr=6. Statistik: one-way Anova mit post-hoc Test nach Dunn's. Signifikante Unterschiede (p < 0,05) werden durch Buchstaben angegeben.

**Tabelle 3E**

| Senkung der durch den Bitterstoff Theobromin ausgelösten Sekretion durch den Bittermaskierer Homoeriodictyol. | | |
|---|---|---|
| **Testsbstanz** | **T/C [%]** | **SEM** |
| 0.3 mM Theobromin | 20.92 | 3.37 a |
| 0.3 mM Theobromin + 0.003 mM HED | 21.56 | 2.33 a |
| 0.3 mM Theobromin + 0.03 mM HED | 13.79 | 3.50 a |
| 0.3 mM Theobromin + 0.3 mM HED | -16.92 | 3.27 b |

In der folgenden **Tabelle 3F** ist die Protonensekretion in HGT-1 Zellen nach 10 minütiger Stimulierung durch 0.3 mM Theobromin oder 0.3 mM Theobromin in Kombination mit Eriodictyol in unterschiedlichen Konzentrationen wiedergegeben. Die Daten sind dargestellt als Mittelwert und mittlere Standardabweichung, n=3, tr=6. Statistik: one-way Anova mit post-hoc Test nach Dunn's. Signifikante Unterschiede (p < 0,05) werden durch Buchstaben angegeben.

**Tabelle 3F**

| Senkung der durch den Bitterstoff Theobromin ausgelösten Sekretion durch den Bittermaskierer Eriodictyol. | | |
|---|---|---|
| **Testsubstanz** | **T/C [%]** | **SEM** |
| 0.3 mM Theobromin | 47.64 | 5.28 a |

(fortgesetzt)

| Senkung der durch den Bitterstoff Theobromin ausgelösten Sekretion durch den Bittermaskierer Eriodictyol. | | |
|---|---|---|
| Testsubstanz | T/C [%] | SEM |
| 0.3 mM Theobromin + 0.03 mM Eriodictyol | 23.95 | 4.17 b |
| 0.3 mM Theobromin + 0.3 mM Eriodictyol | -42.86 | 9.73 c |

In der folgenden **Tabelle 3G** ist die Protonensekretion in HGT-1 Zellen nach 10 minütiger Stimulierung durch 0.3 mM Theobromin oder 0.3 mM Theobromin in Kombination mit Matairesinol in unterschiedlichen Konzentrationen wiedergegeben. Die Daten sind dargestellt als Mittelwert und mittlere Standardabweichung, n=3, tr=6. Statistik: one-way Anova mit post-hoc Test nach Dunn's. Signifikante Unterschiede (p < 0,05) werden durch Buchstaben angegeben.

**Tabelle 3G**

| Senkung der durch den Bitterstoff Theobromin ausgelösten Sekretion durch den Bittermaskierer Matairesinol. | | |
|---|---|---|
| Testsubstanz | T/C [%] | SEM |
| 0.3 mM Theobromin | 34.12 | 2.86 a |
| 0.3 mM Theobromin + 0.03 mM Matairesinol | 23.41 | 2.56 b |
| 0.3 mM Theobromin + 0.3 mM Matairesinol | -18.25 | 3.39 c |

In der folgenden Tabelle 3H ist die prozentuale Steigerung der Protonensekretion in HGT-1 Zellen im Vergleich zu unbehandelten Kontrollen nach 10 minütiger Stimulierung durch 0.3 mM Theobromin oder 0.3 mM Theobromin in Kombination mit Lariciresinol in unterschiedlichen Konzentrationen wiedergegeben. Die Daten sind dargestellt als Mittelwert und mittlere Standardabweichung, n=3, tr=6. Statistik: one-way Anova mit post-hoc Test nach Dunn's. Signifikante Unterschiede (p < 0,05) werden durch Buchstaben angegeben.

**Tabelle 3GH**

| Senkung der durch den Bitterstoff Theobromin ausgelösten Sekretion durch den Bittermaskierer Lariciresinol. | | |
|---|---|---|
| Testsubtanz | T/C [%] | SEM |
| 0.3 mM Theobromin | 30.81 | 3.44 a |
| 0.3 mM Theobromin + 0.03 mM Lariciresinol | 31.73 | 5.80 a |
| 0.3 mM Theobromin + 0.3 mM Lariciresinol | 17.55 | 3.67 a (ttest p<0.05) |

**Beispiel 4**

Sensorische Beurteilung: Bitter-Reduzierung einer bitter schmeckenden Lösung

Die Ergebnisse der folgenden **Tabelle 4** wurden wie in EP 2,517,574 beschrieben ermittelt: Um die Reduzierung (d.h. die Maskierung oder Verminderung) des Bitter-Eindrucks in einer Probe zu quantifizieren, wurde die Bitterkeit des in bestimmter Konzentration vorliegenden bitter schmeckenden Stoffes (Bitterstoff) von einer Expertengruppe jeweils mit einer Probe verglichen, die den in der gleichen Konzentration vorliegenden bitter schmeckenden Stoffes und zusätzlich die jeweils angegebene Menge einer (hinsichtlich der Fähigkeit zur Bitter-Reduzierung) zu beurteilenden Substanz enthielt (Einstufung: 1 [nicht bitter] bis 10 [extrem bitter]). Für die Auswertung, d.h. die Berechnung der Reduktion (in %) des Bittereindrucks wurden jeweils die Durchschnittswerte der Einschätzungen der Expertengruppe für die Lösung des bitter schmeckenden Stoffes und die zu vergleichende, den bitter schmeckenden Stoff und eine zu beurteilende Substanz enthaltende Lösung verwendet. Die Signifikanzberechnung erfolgte über den Student t-Test mit gepaarten Daten bei zweiseitiger Betrachtung.

**Tabelle 4**

| Sensorische Beurteilung | | | | |
|---|---|---|---|---|
| **Bitterstoff** | **Bitterkeit Bitterstoff alleine** | **Testsubstanz** | **Bitterkeit Bitterstoff + Testsubstanz** | **% Reduktion des Bitter-Eindrucks** |
| Coffein (500 ppm) | 6,3±1,4 | Homoeriodictyol (100 ppm) | 4,5±1,4 | 28% (p<0,001) |
| Theobromin (300 ppm) | 4,3±1,8 | Homoeriodictyol (100 ppm) | 3,7±1,3 | 12,5% |
| Coffein (500 ppm) | 6,7±1,1 | Eriodictyol (100 ppm) | 3,5±0,7 | 47% (p<0,0005) |
| Theobromin (300 ppm) | 3,8±1,8 | Eriodictyol (100 ppm) | 2,4±1,2 | 36% (p<0,01) |
| Coffein (500 ppm) | 4,1±0,9 | Matairesinol (25 ppm) | 2,7±1,77 | 35,5 % (p<0,05) |
| Theobromin (300 ppm) | 4,5±2,01 | Matairesinol (25 ppm) | 3,4±1,49 | 24,7% |
| Coffein (500 ppm) | 4,7±1,11 | Lariciresinol (25 ppm) | 2,9±1,41 | 38,6% (p<0,05) |
| Theobromin (300 ppm) | 4,2±1,65 | Lariciresinol (25 ppm) | 3,7±2,24 | 10,7% |

**Patentansprüche**

1. In vitro-Verfahren zur Identifizierung von Bitterstoffen und bittermodulierenden Stoffen, bei dem man Testsubstanzen mit isolierten Magenzellen oder Magentumor-Zelllinien in Kontakt bringt und die dadurch hervorgerufene Änderung des intrazellularen pH-Wertes und/oder die zellulare Protonensekretion bestimmt.

2. Verfahren nach Anspruch 1, **dadurch gekennzeichnet, dass** man Bitterstoffe, bitter-maskierende oder bitter-verstärkende Stoffe identifiziert.

3. Verfahren nach Anspruch 1, **dadurch gekennzeichnet, dass** man Zelllinien einsetzt, die ausgewählt sind aus der Gruppe die gebildet wird von HGT-1, MKN-45, AGS, KATO3, SNU16, und SNU5.

4. Verfahren nach mindestens einem der Ansprüche 1 bis 3, **dadurch charakterisiert,** dass man zur Identifizierung von bitter-maskierenden Stoffen solche Testsubstanzen auswählt, bei denen gilt

$$T/C (bekannter\ Bitterstoff) - T/C (bekannter\ Bitterstoff + Testsubstanz) > 0$$

wobei T/C für den Wert der Protonensekretion in Prozent steht und außerdem die Bedingung besteht, dass die relative Differenz zwischen den beiden Werten mindestens 10 % des größeren Wertes ausmacht.

5. Verfahren nach mindestens einem der Ansprüche 1 bis 3 , **dadurch charakterisiert,** dass man zur Identifizierung von bitter-verstärkenden Stoffen solche Testsubstanzen auswählt, bei denen gilt

$$T/C (bekannter\ Bitterstoff) - T/C (bekannter\ Bitterstoff + Testsubstanz) < 0$$

wobei T/C für den Wert der Protonensekretion in Prozent steht und außerdem die Bedingung besteht, dass die relative Differenz zwischen den beiden Werten mindestens 10 % des größeren Wertes ausmacht,

6. Verfahren nach mindestens einem der Ansprüche 1 bis 3, **dadurch charakterisiert,** dass man zur Identifizierung von Bitterstoffen solche Testsubstanzen auswählt, bei denen gilt

$$T/C \text{ (Kontrolle)} - T/C \text{ (Testsubstanz)} < 0$$

wobei T/C für den Wert der Protonensekretion in Prozent steht und außerdem die Bedingung besteht, dass die relative Differenz zwischen den beiden Werten mindestens 10 % des größeren Wertes ausmacht.

7. Verfahren nach mindestens einem der Ansprüche 1 bis 5, **dadurch gekennzeichnet, dass** man bekannte Bitterstoff einsetzt, die ausgewählt sind aus der Gruppe, die gebildet wird von Stoffen, die in der Lage sind, einen oder mehrere der Bitter-Rezeptor-Typen TAS2R1, TAS2R3, TAS2R7, TAS2R16, TAS2R30, TAS2R38, TAS2R40 und TAS2R50 zu aktivieren.

8. Verfahren nach Anspruch 7, **dadurch gekennzeichnet, dass** die Bitter-Agonisten ausgewählt sind aus der Gruppe, die gebildet wird von Xanthinalkaloiden, Alkaloiden, phenolischen Glycosiden, Flavanoidglycosiden, hydrolisierbaren und nichthydrolysierbaren Tanninen, anderen Polyphenolen, bitteren Isothiocyanaten oder abgeleiteten Stoffen, terpenoiden Bitterstoffen, pharmazeutischen Wirkstoffen, Denatoniumbenzoat oder anderen Denatoniumsalzen, Sucraloseoctaacetat, Harnstoff, Aminosäuren und Peptiden sowie deren Gemischen.

9. Verfahren nach mindestens einem der Ansprüche 1 bis 8, **dadurch gekennzeichnet, dass** man die Änderung des intrazellulären pH-Wertes und/oder der Protonensekretion der Zellen spektrometrisch unter Einsatz eines Fluoreszenzfarbstoffes misst.

10. Verfahren nach mindestens einem der Ansprüche 1 bis 9, **dadurch gekennzeichnet, dass** man die Änderung des intrazellulären pH-Wertes und/oder der Protonensekretion der Zellen über einen Zeitraum von 1 bis 30 min misst.

11. Verfahren nach mindestens einem der Ansprüche 1 bis 10, **dadurch gekennzeichnet, dass** man die (potentiellen) Bitterstoffe in Mengen von 50 bis 150 μl einsetzt.

12. Verfahren nach mindestens einem der Ansprüche 1 bis 11, **dadurch gekennzeichnet, dass** man die Testsubstanzen in Mengen von 0,1 bis 3.000 μM einsetzt.

13. Verfahren nach mindestens einem der Ansprüche 1 bis 12, **dadurch gekennzeichnet, dass** man zur Identifizierung eines bittermodulierenden Stoffes

(a) eine einheitliche Kultur eines Zellsystems ausgewählt aus der Gruppe, die gebildet wird von isolierten Magenzellen oder Magentumor-Zelllinien, zur Verfügung stellt und diese in zwei Proben teilt,
(b) zur ersten Probe einen bekannten Bitterstoff gibt,
(c) zur zweiten Probe den gleichen Bitterstoff aus (b) sowie mindestens eine Testsubstanz gibt,
(d) die Änderung des intrazellulären pH-Wertes und/oder der Protonensekretion der Zellen über die Experimentalzeit misst,
(e) nach Ablauf der Experimentalzeit die Differenz zwischen der Änderung des intrazellulären pH-Wertes und/oder der Protonensekretion der Zellen der ersten und der zweiten Probe bildet
(f) die Testsubstanzen auswählt, bei denen die Differenz entweder positiv oder negativ ist und die relative Differenz zwischen den beiden Werten mindestens 10 % des größeren Wertes ausmacht,
(g) und gegebenenfalls die so ermittelten Testsubstanzen anschließend einer sensorischen Beurteilung unterwirft.

14. Verfahren nach mindestens einem der Ansprüche 1 bis 12, **dadurch gekennzeichnet, dass** man zur Identifizierung eines Bitterstoffes

(a) eine einheitliche Kultur eines Zellsystems ausgewählt aus der Gruppe, die gebildet wird von isolierten Magenzellen oder Magentumor-Zelllinien, zur Verfügung stellt und diese in zwei Proben teilt,
(b) zur ersten Probe eine Kontrolllösung ohne Testsubstanz,
(c) zur zweiten Probe eine Testsubstanz gibt,
(d) die Änderung des intrazellulären pH-Wertes und/oder der Protonensekretion der Zellen über die Experimentalzeit misst,

(e) nach Ablauf der Experimentalzeit die Differenz zwischen der Änderung des intrazellulären pH-Wertes und/oder der Protonensekretion der Zellen der ersten und der zweiten Probe bildet

(f) die Testsubstanzen auswählt, bei denen die Differenz negativ ist und die relative Differenz zwischen den beiden Werten mindestens 10 % des größeren Wertes ausmacht,

(g) und gegebenenfalls die so ermittelten Testsubstanzen anschließend einer sensorischen Beurteilung zur Bestimmung der Bitterwirkung unterwirft.

**Europäisches Patentamt**
**European Patent Office**
**Office européen des brevets**

## EUROPÄISCHER RECHERCHENBERICHT

Nummer der Anmeldung

EP 13 15 5658

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WO 03/031604 A1 (UNIV CALIFORNIA [US]; ROZENGURT JUAN E [US]; PHLEGER COURTNEY S W EM []) 17. April 2003 (2003-04-17) * Seite 35, Absatz 2-3; Ansprüche 18-21; Beispiele 4,5 * | 1-14 | INV. G01N33/50 C07K14/705 |
| Y,D | JESSICA WALKER ET AL: "Identification of Beer Bitter Acids Regulating Mechanisms of Gastric Acid Secretion", JOURNAL OF AGRICULTURAL AND FOOD CHEMISTRY, Bd. 60, Nr. 6, 15. Februar 2012 (2012-02-15), Seiten 1405-1412, XP055062069, ISSN: 0021-8561, DOI: 10.1021/jf204306z * Zusammenfassung * * Seite 1406, rechte Spalte, Absatz 2 * * Seite 1409, linke Spalte, Absatz 3 - Seite 1410, linke Spalte, Absatz 1 * | 1-14 | |
| Y | S. V. WU: "Genomic organization, expression, and function of bitter taste receptors (T2R) in mouse and rat", PHYSIOLOGICAL GENOMICS, Bd. 22, Nr. 2, 14. Juli 2005 (2005-07-14), Seiten 139-149, XP055062070, ISSN: 1094-8341, DOI: 10.1152/physiolgenomics.00030.2005 * Seite 142, rechte Spalte, letzter Absatz - Seite 143, linke Spalte, Absatz 2 *  -/-- | 1-14 | **RECHERCHIERTE SACHGEBIETE (IPC)** G01N C07K |

Der vorliegende Recherchenbericht wurde für alle Patentansprüche erstellt

| Recherchenort | Abschlußdatum der Recherche | Prüfer |
|---|---|---|
| Den Haag | 7. Mai 2013 | Wiesner, Martina |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 13 15 5658

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y,D | WU S V ET AL: "Expression of bitter taste receptors of the T2R family in the gastrointestinal tract and enteroendocrine STC-1 cells", PROCEEDINGS OF THE NATIONAL ACADEMY OF SCIENCES, NATIONAL ACADEMY OF SCIENCES, US, Bd. 99, Nr. 4, 19. Februar 2002 (2002-02-19), Seiten 2392-2397, XP002299242, ISSN: 0027-8424, DOI: 10.1073/PNAS.042617699 * das ganze Dokument * ----- | 1-14 | |
| A | C. STERNINI: "Taste Receptors in the Gastrointestinal Tract. IV. Functional implications of bitter taste receptors in gastrointestinal chemosensing", AMERICAN JOURNAL OF PHYSIOLOGY: GASTROINTESTINAL AND LIVER PHYSIOLOGY, Bd. 292, Nr. 2, 5. Oktober 2006 (2006-10-05), Seiten G457-G461, XP055062071, ISSN: 0193-1857, DOI: 10.1152/ajpgi.00411.2006 * das ganze Dokument * ----- | 1-14 | RECHERCHIERTE SACHGEBIETE (IPC) |
| A | S. JANSSEN ET AL: "Bitter taste receptors and -gustducin regulate the secretion of ghrelin with functional effects on food intake and gastric emptying", PROCEEDINGS OF THE NATIONAL ACADEMY OF SCIENCES, Bd. 108, Nr. 5, 18. Januar 2011 (2011-01-18), Seiten 2094-2099, XP055062072, ISSN: 0027-8424, DOI: 10.1073/pnas.1011508108 * das ganze Dokument * ----- | 1-14 | |

Der vorliegende Recherchenbericht wurde für alle Patentansprüche erstellt

| Recherchenort | Abschlußdatum der Recherche | Prüfer |
|---|---|---|
| Den Haag | 7. Mai 2013 | Wiesner, Martina |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

...............................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 13 15 5658

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-05-2013

| Im Recherchenbericht angeführtes Patentdokument | Datum der Veröffentlichung | Mitglied(er) der Patentfamilie | Datum der Veröffentlichung |
|---|---|---|---|
| WO 03031604 A1 | 17-04-2003 | CA 2463553 A1 | 17-04-2003 |
| | | EP 1442117 A1 | 04-08-2004 |
| | | JP 2005522187 A | 28-07-2005 |
| | | US 2007059688 A1 | 15-03-2007 |
| | | WO 03031604 A1 | 17-04-2003 |

Für nähere Einzelheiten zu diesem Anhang : siehe Amtsblatt des Europäischen Patentamts, Nr.12/82

EPO FORM P0461

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004029087 A1
- WO 2008057470 A1
- EP 2517574 A

**In der Beschreibung aufgeführte Nicht-Patentliteratur**

- **MEYERHOF.** *Reviews of Physiology, Biochemistry and Pharmacology,* 2005, vol. 154, 37-72
- **DIKSHIT, M.** A flowcytometric method for evaluation of acid secretion from isolated rat gastric mucosal cells. *J Pharmacol Toxicol Methods,* 2001, vol. 45, 47-53
- **MATSUNO, K. ; TOMITA, K. ; OKABE, S.** Wine stimulates gastric acid secretion in isolated rabbit gastric glands via two different pathways. *Aliment Pharmacol Ther,* vol. 16 (2), 107-14
- **VILA-PETROFF, M. ; MUNDIHA-WEILENMANN, C. ; LEZCANO, N. ; SNABAITIS, A. K. ; HUERGO, M. A. ; VALVERDE, C. A. ; AVKIRAN, M. ; MATTIAZZI, A.** Ca(2+)/calmodulin-dependent protein kinase II contributes to intracellular pH recovery from acidosis via Na(+)/H(+) exchanger activation. *J Mol Cell Cardiol,* 2010, vol. 49, 106-12
- **SWIETACH, P. ; ROSSINI, A. ; SPITZER, K. W. ; VAUGHAN-JONES, R. D.** H+ ion activation and inactivation of the ventricular gap junction: a basis for spatial regulation of intracellular pH. *Circ Res,* 2007, vol. 100, 1045-54
- **LISZT et al.** *J. Agric. Food Chem.,* 2012, vol. 60 (28), 7022-7030
- **WALKER et al.** *J. Agric. Food Chem.,* 2012, vol. 60 (6), 1405-1412
- **RUBACH et al.** *J. Agric. Food Chem.,* 2010, vol. 58, 4153-61

- **BEHRENS et al.** *Physiology & Behavior,* 2011, vol. 105 (1), 4-13
- *Chem Rev.,* 2010, vol. 110 (5), 2709-2728
- **DING, X. ; DENG, H. ; WANG, D. ; ZHOU, J. ; HUANG, Y. ; ZHAO, X. ; YU, X. ; WANG, M. ; WANG, F. ; WARD, T.** Phospho-regulated ACAP4-Ezrin interaction is essential for histamine-stimulated parietal cell secretion. *J Biol Chem,* 2010, vol. 285, 18769-80
- **MALTE RUBACH, R. L. ; ELISABETH SEEBACH ; MARK M. SOMOZA ; THOMAS HOFMANN ; SOMOZA, A. V.** *Mol Nutr Food Res. in press,* 2011
- **RUBACH, M. ; LANG, R. ; HOFMANN, T. ; SOMOZA, V.** *Ann N Y Acad Sci,* 2008, vol. 1126, 310-4
- **RUBACH, M. ; LANG, R. ; SKUPIN, C. ; HOFMANN, T. ; SOMOZA, V.** *J Agric Food Chem,* 2010, vol. 58, 4153-61
- **WEISS, C. ; RUBACH, M. ; LANG, R. ; SEEBACH, E. ; BLUMBERG, S. ; FRANK, O. ; HOFMANN, T. ; SOMOZA, V.** *J Agric Food Chem,* 2010, vol. 58, 1976-85
- **LISZT, K. I. ; WALKER, J. ; SOMOZA, V.** *J Agric Food Chem,* 2012
- **WALKER, J. ; HELL, J. ; LISZT, K. I. ; DRESEL, M. ; PIGNITTER, M. ; HOFMANN, T. ; SOMOZA, V.** *J Agric Food Chem,* 2012, vol. 60, 1405-12